(19) Europäisches Patentamt
European Patent Office
Office européen des brevets

(11) Publication number: **0 227 431 A1**

(12) **EUROPEAN PATENT APPLICATION**

(21) Application number: 86309873.7

(22) Date of filing: 17.12.86

(51) Int. Cl.4: C 07 D 333/38
C 07 D 333/68, C 07 D 231/40
C 07 D 213/79, C 07 D 213/80
A 61 K 31/38, A 61 K 31/415
A 61 K 31/44, C 07 D 333/00
C 07 D 231/00, C 07 D 213/00

(30) Priority: 17.12.85 GB 8531019

(43) Date of publication of application: 01.07.87 Bulletin 87/27

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Outred, Dennis James Beecham Pharmaceuticals
Great Burgh Yew Tree Bottom Road
Epsom Surrey KT18 5XO(GB)

(74) Representative: Russell, Brian John et al,
European Patent Attorney Beecham Pharmaceuticals
Great Burgh Yew Tree Bottom Road
Epsom Surrey KT18 5XQ(GB)

(54) **Heterocyclic amides.**

(57) A compound of formula (I):

(I) 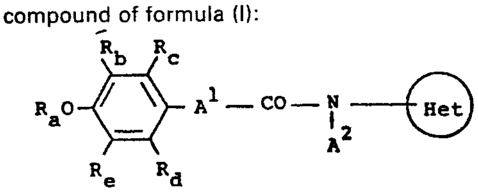

or a salt, ester, amide or solvate thereof, in which,

$R_a$ represents hydrogen, substituted or unsubstituted alkyl or an acyl group;

$R_b$ and $R_c$ are independently hydrogen or a substituted or unsubstituted aryl group or a group $-OR_a$; or $R_b$ and $R_c$ together with the carbon atoms to which they are attached form a substituted or unsubstituted carbocyclic group;

$R_d$ and $R_e$ are independently hydrogen, substituted or unsubstituted aryl or a group $-OR_a$; or $R_d$ and $R_e$ together with the carbon atoms to which they are attached form a substituted or unsubstituted carbocyclic group;

$A^1$ represents an alkylene or an alkenylene group;

$A^2$ represents hydrogen, substituted or unsubstituted alkyl or an aralkyl group substituted or unsubstituted in the aryl moiety;

and Het represents a substituted or unsubstituted heterocyclic group; a process for preparing such a compound, a pharmaceutical composition containing such a compound and the use of such compounds and compositions in medicine.

EP 0 227 431 A1

TITLE MODIFIED
see front page

B1969

NOVEL COMPOUNDS

This invention relates to novel heterocyclic amides, to processes for preparing them, to pharmaceutical compositions containing them and their use in medicine.

British Patent No 1446141 discloses compounds of formula (A):

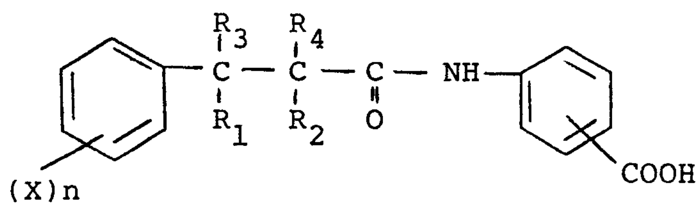

(A)

in which $R_1$ and $R_2$ are each individually a hydrogen atom or an alkyl group having from 1-4 carbon atoms, $R_3$ and $R_4$ are each a hydrogen atom or may be combined together to form another carbon to carbon bond, each X atom is individually a hydroxyl group, a halogen atom, an alkyl group having from 1 to 4 carbon atoms or an alkoxy group having 1 to 4 carbon atoms, and n is an integer from 1 to 3; or the group $(X)_n$ is either the group $-O-CH_2-O-$ or the group $-(O)_a-(CH_2)_b-(O)_c-$ wherein a and c are each either 0 or 1 and b is an integer of from 2 to 10 wherein the said group is attached to adjacent carbon atoms of the benzene ring to form a ring.

The compounds of formula (A) are said to inhibit disruption of mast cells and subsequent release of chemical mediators which cause allergic reactions.

Japanese Published Patent Application No. 97946/85

discloses compounds of formula (B).

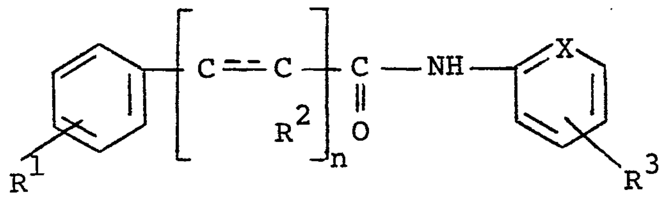

(B)

in which

$R^1$ = 5-15C linear or branched alkyl or alkoxy; 5-15C linear or branched alkenyl or alkenyloxy in the 2- or 3-position of the benzene ring; or 13-15C linear or branched alkenyl or 5-15C linear or branched alkenyloxy in the 4-position of the benzene ring;

$R^2$ = H or methyl;

$R^3$ = OH, carboxy, carboxymethyl, carboxymethoxy, 2-6C linear or branched alkoxycarbonyl, or 3-7C linear or branched alkoxycarbonylmethyl or alkoxycarbonylmethoxy;

X = - CH = or nitrogen;

n = 0,1 or 2; and

-- means optional double bond (E or Z).

These compounds are disclosed as leukotriene-antagonists and 5a-reductase and phospholipase-inhibitors.

It is generally recognised that the slow reacting substance of anaphylaxis (SRS-A, now known to be a mixture of the leukotrienes $C_4$, $D_4$ and $E_4$) can be

released from cells other than the mast cell, and in particular from the eosinophil, and that mast cell stablising drugs such as disodium cromoglycate are unable to inhibit this release. A compound that is able to stabilise sensitised mast cells to antigen challenge and inhibit the enzymes involved in the release and subsequent oxidative metabolism of arachidonic acid to 5-hydroperoxyeicosatetraenoic acid, and thus to SRS-A, should therefore be a potentially valuable drug for the treatment of asthma and other diseases in which SRS-A and other mast cell products are involved. Typical diseases include asthma, rhinitis, hayfever, allergic eczema and psoriasis. Compounds produced via the oxidative metabolism of arachidonic acid are also involved in inflammatory diseases such as rheumatoid arthritis and compounds of the type described here should also be of value in the diseases of this type.

We have now discovered a new class of heterocyclic amides which inhibit the antigen-induced release of histamine from peritoneal mast cells in addition to inhibiting phospholipase $A_2$, one of the enzymes responsible for the release of arachidonic acid from its phospholipid store. These compounds also inhibit 5-lipoxygenase, the enzyme involved in the oxidative metabolism of arachidonic acid to 5-hydroperoxy-eicosatretraenoic acid, the primary precursor to SRS-A. These compounds are thus of value in the prophylaxis and treatment of diseases whose symptoms are controlled by mast cell derived mediators such as histamine and by those metabolites of arachidonic acid derived via initial peroxidation at C-5, by preventing the formation of bronchoconstrictor and inflammatory mediators.

Accordingly the present invention provides a compound of formula (I):

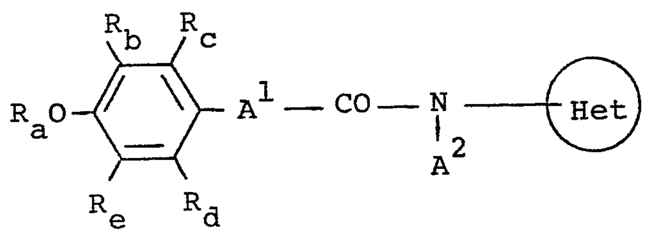

(I)

or a salt, ester, amide or solvate thereof, in which, $R_a$ represents hydrogen, substituted or unsubstituted alkyl or an acyl group;

$R_b$ and $R_c$ are independently hydrogen or a substituted or unsubstituted aryl group or a group $-OR_a$; or $R_b$ and $R_c$ together with the carbon atoms to which they are attached form a substituted or unsubstituted carbocyclic group;

$R_d$ and $R_e$ are independently hydrogen, substituted or unsubstituted aryl or a group $-OR_a$; or $R_d$ and $R_e$ together with the carbon atoms to which they are attached form a substituted or unsubstituted carbocyclic group;

$A^1$ represents an alkylene or an alkenylene group;

$A^2$ represents hydrogen, substituted or unsubstituted alkyl or an aralkyl group substituted or unsubstituted in the aryl moiety;

and Het represents a substituted or unsubstituted heterocyclic group.

Suitable heterocyclic groups Het, include substituted or unsubstituted single or fused ring heterocyclic groups having 4 to 7, preferably 5- or 6-, atoms in each ring, there being up to four hetero atoms selected

from oxygen, nitrogen or sulphur in each ring, and at least one hetero atom in at least one of the rings.

Suitable optional substituents for any heterocyclic group include up to three substituents selected from the list consisting of: halo, alkyl, alkoxy, haloalkyl, hydroxy, amino, carboxy or substituted or unsubstituted aryl.

When used herein the term 'aryl' includes phenyl and naphthyl optionally substituted with up to five, preferably up to three, groups selected from halogen, alkyl, phenyl, alkoxy, haloalkyl, hydroxy, amino, nitro, carboxy, alkoxycarbonyl, alkoxycarbonylalkyl, alkylcarbonyloxy, or alkylcarbonyl groups.

Suitable optional substitutents for any alkyl group or alicyclic group include those indicated above in relation to the aryl group.

Favourable heterocyclic groups Het, include groups of formula

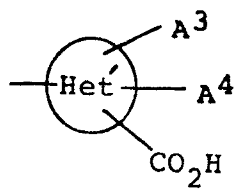

wherein Het′ represents a 5- or 6- membered single ring having up to 4 hetero atoms in the ring selected from oxygen, nitrogen or sulphur; and
$A^3$ and $A^4$ are independently hydrogen, alkyl, substituted or unsubstituted aryl or $A^3$ and $A^4$ together with the atoms to which they are attached form a saturated or unsaturated carbocyclic group.

Preferred heterocyclic groups Het or Het′ contain up to two heteroatoms selected from oxygen, nitrogen or sulphur in each ring.

Suitable carbocyclic groups include single or fused alicyclic or aryl rings, each ring consisting of 5- or 6- carbon atoms.

Suitably, only one of $R_b$, $R_c$, $R_d$ or $R_e$ represents a group $-OR_a$.

Suitable acyl groups represented by $R_a$ are those of formula -CO-R wherein R represents substituted or unsubstituted alkyl or substituted or unsubstituted aryl or aralkyl substituted or unsubstituted in the aryl ring.

Preferably, R represents alkyl or a substituted or unsubstituted benzene ring.

Preferred acyl groups are those wherein R is $C_{1-6}$ alkyl or benzene or $C_{1-6}$ alkyl-substituted benzene.

Preferably, $R_c$ represents hydrogen.

Preferably, $R_d$ represents hydrogen.

Most preferably $R_c$ and $R_d$ both represent hydrogen and one of $R_b$ or $R_e$ represents hydrogen and the remaining variable of $R_b$ and $R_e$ represents a group $-OR_a$.

In a preferred aspect the present invention provides a group of compounds, falling within the scope of formula (I), of general formula (II):

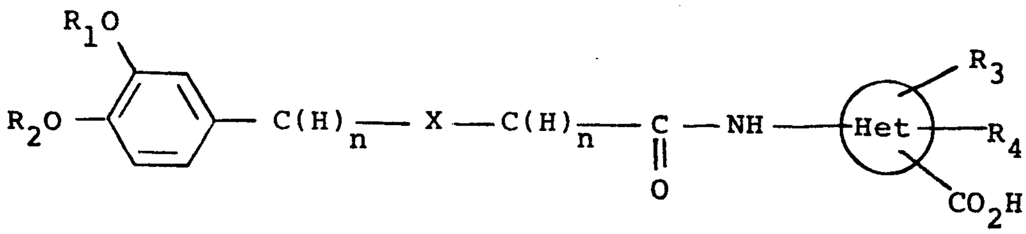

(II)

or a salt, ester, amide or solvate thereof,

in which

Het is as defined above in relation to formula (I),

either n is 1 and X is a double bond,

or n is 2 and X is a single bond,

$R_1$ and $R_2$ are indendently hydrogen, $C_1$-$C_6$ alkyl or acyl, $R_3$ and $R_4$ are independently hydrogen, $C_1$-$C_6$ alkyl, substituted or unsubstituted aryl, or $R^3$ and $R^4$, together with the atoms to which they are attached, form a saturated or unsaturated carbocyclic ring.

X is preferably a double bond (and n is 1). When X is a double bond, the hydrocarbon chain containing X may have the (E) or (Z) absolute configuration, but the (E) configuration is preferred.

Examples of suitable 5-membered hetorocyclic rings are thiophene, furan, oxazole, isoxazole, thiazole, isothiazole, pyrrole, pyrazole and the isomeric triazoles.

Suitable 6-membered heterocyclic rings are pyridine, pyridazine, pyrimidine, pyrazine and the isomeric triazines.

A preferred heterocyclic group is thiophene.

$R_1$ and $R_2$ are suitably hydrogen or a methyl, acetyl, propionyl, trimethylacetyl or a (4-methyl)benzoyl group.

$R_3$ and $R_4$ are suitably hydrogen; or $R_3$ and $R_4$, together with the atoms to which they are attached, from a fused benzo, tetrahydrobenzo or cyclopentyl ring.

The group $-CO_2H$ is preferably attached to the heterocyclic ring in a position ortho to the extranuclear nitrogen atom of the amido group.

The compounds of formula (I) or their salts, esters, amides or solvates are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 90% and more preferably 95%. One preferred pharmaceutically acceptable form is the crystalline form. In the case of salts, ester, amides and solvates the additional ionic esteryfing, amide-forming and solvent moieties must also be non-toxic.

Suitable pharmaceutically acceptable salts of the compounds of formula (I) are pharmaceutically acceptable salts of carboxy groups or hydroxy groups.

Examples of a pharmaceutically acceptable salt of a carboxy group of a compound of formula (I) include alkali metal and alkaline earth metal salts, such as sodium, potassium and magnesium salts; and salts with ammonia, organic bases and amino compounds. Also when a basic nitrogen atom is present the compounds of the formula (I) can form acid addition salts with the conventional pharmaceutical acids, for example, maleic, hydrochloric, hydrobromic, phosphoric, acetic, fumaric, salicylic, citric, lactic, mandelic, tartaric, succinic, benzoic, ascorbic and methanesulphonic acids.

Suitable pharmaceutically acceptable salts of hydroxyl groups include metal salts, especially alkali metal salts such as sodium and potassium salts.

Suitable pharmaceutically acceptable esters of compounds of formula (I) include esters of carboxy groups and hydroxy groups.

Suitable pharmaceutically acceptable esters are in-vivo hydrolysable esters of carboxy groups and hydroxy groups.

Suitable in-vivo hydrolysable esters are those used conventionally in the art.

Suitable esters of carboxyl groups are the $C_{1-6}$ alkyl esters, especially methyl, ethyl, or t-butyl esters.

Suitable esters of hydroxyl groups include those provided by $C_{1-6}$ alkyl carboxylic acids.

Suitable pharmaceutically acceptable amides include amides of formula -CO. $NR^s$ $R^t$ wherein $R^s$ and $R^t$ each

independently represent hydrogen or $C_{1-6}$ alkyl; or $R^s$ and $R^t$ together with the nitrogen to which they are attached represent a saturated 5- or 6- membered ring.

Suitable pharmaceutically acceptable solvates of a compound of formula (I) includes hydrates.

Salts, esters, amides or solvates of compounds of formula (I) which are not pharmaceutically acceptable also form an aspect of the invention since they may be used as intermediates to prepare pharmaceutically acceptable compounds.

When used herein the term ''halogen'' refers to fluorine, chlorine, bromine and iodine; preferably chlorine.

When used herein the term ''alkyl'', ''alkenyl'', ''alkynyl'' or ''alkoxy'' relates to groups having straight or branched chains containing up to 12 carbon atoms.

Suitable alkyl groups are $C_{1-12}$ alkyl groups especially $C_{1-6}$ alkyl groups e.g. methyl, ethyl, n-propyl, iso-propyl, n-butyl, isobutyl or tert-butyl groups.

Suitable alkenyl groups are $C_{2-12}$ groups especially $C_{2-6}$ alkenyl groups.

Suitable alkynyl groups are $C_{2-12}$ alkynyl groups especially $C_{2-6}$ alkynyl groups.

When used herein the term ''in-vivo'' hydrolysable ester'' relates to a pharmaceutically acceptable ester which readily breaks down in the human or non-human animal body to leave for example in relation to an

in-vivo hydrolysable ester of a carboxy group the free carboxy group or a salt thereof or for example in relation to an in-vivo hydrolysable ester of an hydroxy group, the free hydroxy group, or a salt thereof.

The present invention also provides a process for preparing a compound of formula (I) which comprises reacting a compound of formula (III)

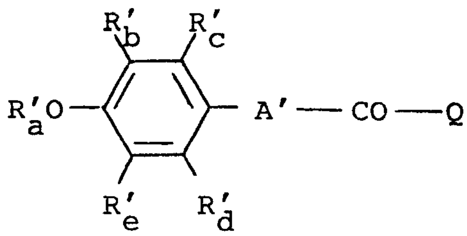

(III)

in which $R'_a$, $R'_b$, $R'_c$, $R'_d$ and $R'_e$ are either $R_a$, $R_b$, $R_c$, $R_d$ or $R_e$ as defined in respect of formula (I) or a group or atom convertible to $R_a$, $R_b$, $R_c$, $R_d$, or $R_e$; A' is as defined in relation to formula (I) and Q is a leaving group; with a compound of formula (IV)

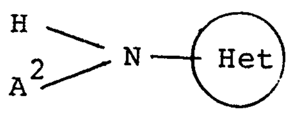

(IV)

in which Het and $A^2$ are as defined in respect of formula (I);

to form a compound of formula (Ia)

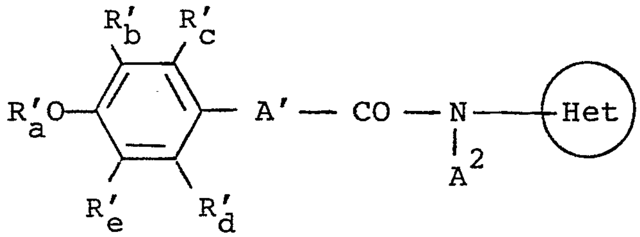

(Ia)

and thereafter where appropriate converting $R'_a$, $R'_b$, $R'_c$, $R'_d$ or $R'_e$ to $R_a$, $R_b$, $R_c$, $R_d$ or $R_e$ respectively, and optionally forming a salt, ester, amide or solvate thereof.

The leaving group Q is typically a halogen such as chloro or bromo, preferably chloro.

The reaction of compounds (III) and (IV) may be effected by stirring together in a solvent such as pyridine at ambient temperatures.

A compound of formula (Ia) may be converted to a compound of formula (I), or one compound of formula (I) converted to another, by interconversion of suitable substituents using appropriate conventional methods.

For example an acyl group represented by $R'_a$ (an $R'_a$ acyl group) may be converted to an $R_a$ hydrogen atom by treating with sodium methoxide in alcohol. A group $-OR'_a$ wherein $R'_a$ is hydrogen may be converted to a group $-OR_a$ wherein $R_a$ is alkyl or acyl by treatment with an appropriate halide (or anhydrides) under conventional alkylation or acylation conditions. In addition a compound in which $A^1$ is an alkenylene group may be converted to one in which $A^1$ is an alkylene group by conventional hydrogenation. Also an ester of a compound of formula (Ia), prepared by conventional

esterification methods, may be hydrolysed to a free carboxyl group with aqueous alkali, and the carboxyl group then converted to a further salt, ester, amide or solvate of a compound of formula (I) by the appropriate conventional procedure.

It will be appreciated that similar conventional procedures may be used to convert a compound of formula (I) to a further compound of formula (I).

Compounds of formula (III) may be prepared from a compound of formula (V)

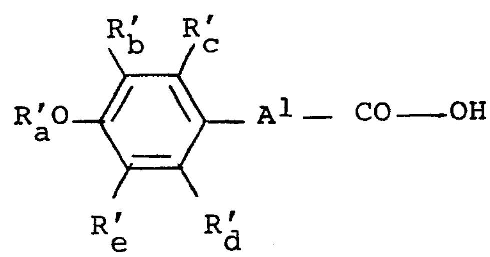

(V)

wherein $R'_a$, $R'_b$, $R'_c$, $R'_d$, $R'_e$ and $A^1$ are as defined in relation to formula (II); by reaction with a halogenating agent.

Suitable halogenating agents are thionyl chloride or, more preferably, oxalyl chloride. The reaction suitably takes place at ambient temperatures in a solvent such as chloroform or dichloromethane, in the presence of an catalytic amount of N,N-dimethylformamide.

If the desired end product is a compound in which for example $R_a$ is hydrogen, then in the compound of formula (V) the hydroxy group $OR_a$ is preferably protected by forming an acyl group, for example by treating with an acyl anhydride, such as acetic anhydride, in the presence of a catalytic amount of sulphuric acid in an

inert solvent such as ether. Deprotection is carried out as described earlier for converting an $R'_a$ acyl to an $R_a$ hydrogen.

Compounds of formula (IV) and (V) are known compounds or may be prepared from known compounds by conventional procedures. For example they may be prepared by using the procedures or analogous procedures to those disclosed in Eur. J. Med. Chem. 1980, 15(5), 413 for compounds of formula (IV) and Chem. Ber. 1878, 11,656 for compounds of formula (V).

The compounds of formula (I) are indicated as active therapeutic agents by their inhibition of phospholipase $A_2$ and 5-lipoxygenase, and by their inhibition of histamine and SRS-A release in the rat passive peritoneal anaphylaxis model.

Accordingly, the present invention provides a pharmaceutically acceptable compound of formula (I) or pharmaceutically acceptable salt, ester, amide or solvate thereof, as an active therapeutic substance.

The present invention further provides a pharmaceutically acceptable compound of the formula (I), or a pharmaceutically acceptable salt, ester, amide or solvate thereof, for use in the treatment or prophylaxis of allergic diseases, such as asthma, hayfever, rhinitis or allergic eczema, or for treatment of inflammatory conditions such as arthritis and psoriasis.

The present invention also provides a pharmaceutical composition comprising a pharmaceutically acceptable compound of the formula (I), or a pharmaceutically acceptable salt, ester, amide or solvate thereof, in

admixture or conjunction with a pharmaceutically acceptable carrier.

In a further aspect the present invention provides the use of a pharmaceutically acceptable compound of formula (I), or a pharmaceutically acceptable salt, ester, amide or solvate thereof, for the manufacture of a medicament for the treatment or prophylaxis of respiratory diseases, such as asthma, hayfever, rhinitis, or allergic eczema, or for the treatment of inflammatory conditions such as arthritis and psoriasis.

Preferably, a pharmaceutical composition of the invention is in unit dosage form and in a form adapted for use in the medical or veterinarial fields. For example, such preparations may be in a pack form accompanied by written or printed instructions for use as an agent in the treatment or prophylaxis of any of the disorders mentioned above.

The suitable dosage range for the compounds of the invention may vary from compound to compound and may depend on the condition to be treated. It will also depend, inter alia, upon the relation of potency to absorbability and the mode of administration chosen. The compound or composition of the invention may be formulated for administration by any route, the preferred route depending upon the disorder for which treatment is required, and is preferably in unit dosage form or in a form that a human patient may administer to himself in a single dosage. Advantageously, the composition is suitable for oral, rectal, topical, parenteral, intravenous or intramuscular administration or through the respiratory tract. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories. Compositions which are especially suitable for administration to the respiratory tract and for topical administration are discussed in more detail below.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable setting agents such as sodium lauryl sulphate.

Solid compositions may be obtained by conventional methods of blending, filling tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible

capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable

additives. Such forms will be presented in unit dose form such as ampoules or disposable injection devices or in multi- dose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

For topical administration, compositions may be presented as an ointment, cream, lotion, gel, aerosol, or skin paint.

By way of example, in any of the preceding formulations a suitable dosage unit might contain 0.01 to 500mgs of active ingredient, more suitably 1 to 500mgs via the oral route, 0.01 to 10mgs via inhalation. The effective dose of compound depends on the particular compound employed, the condition of the patient and on the frequency and route of administration, but in general is in the range of from 0.001 mg/kg/day to 100 mg/kg/day inclusive of the patient's body weight.

For use in treatment of inflammatory disorders a composition of the invention will preferably be in a form suitable for oral administration, for example a tablet or capsule or a sachet containing reconstitutable powder. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

Compounds of this invention and their preparation are illustrated in the following Examples. (Temperatures are in $^{o}C$.)

Preparation 1

4-Acetoxy-3-methoxycinnamic acid

Ferulic acid (4-hydroxy-3-methoxycinnamic acid) (8.10g) was treated with acetic anhydride (15ml) and conc. sulphuric acid (10 drops) with stirring at room temperature. After 5 min dry ether was added and the stirring was continued for a further 3 hr. The mixture was poured onto ice, the product was extracted with ethyl acetate. The solvent extract was washed, dried and evaporated to yield the title compund 9.75g (98%) m.p. 194°.

Preparation 2

4-Acetoxy-3-methoxycinnamoyl chloride

The acid from Preparation 1 (5.00g) was suspended in dichloromethane (50ml) and dimethylformamide (10 drops) and treated with oxalyl chloride (3.00g) at room temperature. After 0.5hr, the clear yellow solution was evaporated under reduced pressure to yield the crude title compound (5.15g) which was used without further purification.

Preparation 3

3,4-Dipropionyloxycinnamic acid

3,4-Dihydroxycinnamic acid (5.00g) was treated with propionic anhydride (20ml) and conc. sulphuric acid (10 drops) as described in Preparation 1. The title compound 3.60g (45%) was isolated by chromatography m.p. 166°.

## Preparation 4

## 3,4-Dipropionyloxycinnamoyl chloride

The acid from Preparation 3 was converted to the acid chloride as described in Preparation 2. Treatment of the acid (2.93g) yielded the title compound 3.01g (96%).

## Preparation 5

## t-Butyl-2-amino-4,5,6,7-tetrahydrobenzo[b]thiophene-3-carboxylate

The title compound was prepared from t-Butylcyanoacetate, sulphur, and cyclohexanone using the method decribed by Perrisin et al. (Eur. J. Med. Chem 1980, 15(5), 413). The title compound was obtained as a thick yellow oil (bp 185%/0.06nm) in 75% yield.

## Preparation 6

## t-Butyl-2-amino-5-methyl-4,5,6,7-tetrahydrobenzo[b]-thiophene-3-carboxylate

The title compound was prepared by the same method as Preparation 6, using 4-methyl-cyclohexanone in place of cyclohexanone. The product was isolated by chromatography on silica gel (dichloromethane-hexane 1:1) as a thick yellow gum. Yield (63%).

Preparation 7

Methyl-3-trifluoro acetamidothiophene-2-carboxylate

Methyl-3-aminothiophene-2-carboxylate (2.50g) was dissolved in trifluoroacetic acid (16ml) and treated with trifluoroacetic anhydride (1.5ml). The mixture was stirred at room temperature for 3 hr. The reaction mixture was poured onto ice, and the solid which precipitated was collected, washed with water and dried mp 74° 2.81g (70%). A sample which was recrystallized from hexane mp 76° was analytically pure.

Preparation 8

Methyl-N-benzyl-3-trifluoroacetamidothiphene-2-carboxylate

The product from Preparation 7 (1.44g) was stirred with potassium carbonate (0.95g) in dry dimethylformamide (15ml) at 70°. Benzylbromide (1.07g) was added dropwise and the reaction stirred at 70° for 2hr. The title compound was isolated by solvent extraction and chromatography on silica gel (ethylacetate-hexane 1:4) as a white solid 1.28g mp 54° (66%) and was analytically pure.

Preparation 9

Methyl-3-benzylaminothiophene-2-carboxylate

The product from Preparation 8 (1.20g) was treated with 7% potassium carbonate in 25ml methanol-water (7:2), and stirred at room temperature for 1 hr. The white solid which had precipitated was collected, washed, and dried to yield the title compound mp 80° 0.82g (85%) which was analytically pure.

Example 1

Methyl 3-[N-(3,4-diacetoxycinnamoyl) amino] thiophene-2-carboxylate

Methyl 3-aminothiophene-2-carboxylate(1.00g, 0.006mol)was dissolved in dry pyridine (10ml) and a solution of 3,4-diacetoxycinnamoyl chloride (1.75g=0.006mol) in pyridine (10ml) was added at room temperature with stirring.

After 3hr. the pyridine was evaporated, and the residue was dissolved in chloroform. The chloroform solution was washed with dilute sodium hydroxide solution, 2M hydrochloric acid and water. The solvent layer was dried ($MgSO_4$), and evaporated to yield a red semi-solid (1.70g).

Chromatography on Kieselgel 60 (Ethylacetate-Hexane 1:1) gave the required product (0.97g) as a pale yellow solid. Recrystallization (EtOAc) gave pure material m.p. 164°C. Yield=39%.
Found C.56.35, H. 4.28, N. 3.50, S. 7.83%
Requires C. 56.57, H. 4.25, N. 3.47, S. 7.95% for $C_{19}H_{17}NO_7S$

In a repeat reaction the 3,4-diacetoxy cinnamoyl chloride was prepared "in situ" from 3,4-diacetoxycinnamic acid (1.57g=0.006ml). The yield of pure compound was 49%.

Example 2

Methyl 3-[N-(4-acetoxy-3-methoxycinnamoyl)-amino]-thiophene-2-carboxylate

Methyl-3-aminothiophene-2-carboxylate (2.00g) and 4-acetoxy-3-methoxycinnamoyl chloride (3.50g) were reacted as described in Example 1.

Crystallization of the crude product from ethylacetate / hexane yielded the title compound 3.62g (76%).

Found C. 57.46, H. 4.51, N. 3.46%
Requires C. 57.59, H. 4.56, N. 3.73% for $C_{18}H_{17}NO_6S$.

Example 3

Ethyl 3-[N-(3,4-diacetoxycinnamoyl)-amino]-pyrazole-4-carboxlate

Ethyl 3-aminopyrazole-4-carboxylate (2.00g) and 3,4-diacetoxycinnamoyl chloride were reacted as described in Example 1.

Chromatography on Kieselgel 60 (Ethylacetate-hexane, 4:6) gave pure title compound 1.65g (32%) m.p. 154$^{O}$

Found C. 56.59, H. 4.72, N. 10.24%
Requires C. 56.85, H. 4.77, N. 10.47% for $C_{19}H_{19}N_3O_7$.

Example 4

Ethyl 2-[N-(3,4-diacetoxycinnamoyl)amino] pyridine-3-carboxylate

Ethyl-2-aminonicotinate (2.30g) and 3,4-diacetoxycinnamoyl chloride (4.01g) were reacted in pyridine (40ml) as described in Example 1. Chromatography of the crude product on Kieselgel 60 (Ethylacetate: Hexane, 7:3) gave pure title compound (3.79g) (66%) m.p. 163$^{O}$.

Found C.60.79, H. 4.83, N. 6.51%
Requires C. 61.16, H. 4.89, N. 6.79% for $C_{21}H_{20}N_2O_7$.

Example 5

Ethyl-2-[N-(3,4-diacetoxycinnamoyl)amino]-4,5,6,7-tetrahydrobenzo - [b]-thiophene-3-carboxylate

A solution of freshly prepared 3,4-diacetoxycinnamoyl chloride (from 3,4-diacetoxycinnamic acid, 5.20g) in toluene (15ml) was added dropwise to a solution of ethyl-2-amino 4,5,6,7-tetrahydrobenzo [b]-thiophene-3-carboxylate (4,52g) in pyridine (25ml). The reaction mixture was stirred and cooled in ice. When the addition was complete the reaction was stirred at ambient temperature for 3hr. Treatment as described in Example 1 yielded a yellow solid (7.95g) m.p. 174° which was crystallized from ethyl acetate to give pure title compound (7.40g) (78%) m.p. 197°.

Found C. 61.52, H. 5.54, N. 3.02, S. 6.81%
Requires C. 61.13, H. 5.34, N. 2.97, S. 6.80% for $C_{24}H_{25}NO_7S$.

Example 6

Methyl-3[N-(3,4-dimethoxycinnamoyl)amino]thiophene-2-carboxylate

A solution of freshly prepared 3,4-dimethoxycinnamoyl chloride (from 3,4-dimethoxycinnamic acid 2.08g) was dissolved in toluene (25ml) and added dropwise to a solution of methyl-3-aminothiophene-2-carboxylate (1.57g) in pyridine (20ml). The reaction was stirred and cooled in ice. When the addition was complete, the mixture was stirred at ambient temperature for 3hr. Treatment of the mixture as described in Example 1 gave the crude product, which was crystallized from ethyl acetate to yield pure title compound (2.51g) (12%) m.p. 150°.

Found C. 58.75, H. 4.89, N. 4.03, S. 9.23%
Requires C. 58.77, H. 4.93, N. 4.03, S. 9.23% for $C_{17}H_{17}NO_5S$.

Example 7

Methyl 3-[N-(3,4-dihydroxycinnamoyl)-amino]thiophene-2-carboxylate

The ester obtained in Example 1 (1.00g) was suspended in methanol (20ml) and stirred at room temperature. Sodium metal (10mg) was added and the reaction was stirred until a clear solution was obtained (1hr). The reaction mixture was diluted with water and acidified with dilute hydrochloric acid. A yellow solid precipitated which was collected, washed and dried (0.68g) m.p. 202°. Recrystallization from ethanol gave pure title compound m.p. 210°(86%)

Found C. 56.08, H. 4.07, N. 4.32, S. 10.32%
Requires C. 56.42, H. 4.10, N. 4.39, S. 10.04% for $C_{15}H_{13}NO_5S$

Example 8

Methyl 3-[N-(4-hydroxy-3-methoxycinnamoyl)-amino]thiophene-2-carboxylate

The product from Example 2 (3.60g) was treated with methanol and sodium metal as described in Example 7. Chromatography on Kieselgel 60 (EtOAc: Hexane, 4:6) gave the title compound 2.00g (56%) m.p. 102° as a crystalline solid containing 0.5mole of ethyl acetate.

Found C. 57.48, H. 5.04, N. 3.54, S. 8.37%
Requires C. 57.28, H. 5.07, N. 3.71, S. 8.50% for $C_{16}H_{15}NO_5S + 0.5.C_4H_8O_2$.

The 'H Nuclear Magnetic Resonance spectrum confirms the presence of 0.5 mole of ethyl acetate in the product.

Example 9

Ethyl 3-[N-(3,4-dihydroxycinnamoyl)amino]pyrazole-4-carboxylate

The diacetyl derivative described in Example 3 (1.50g) was treated with sodium and ethanol as described in Example 7. The crude product was crystallized from ethanol to yield the title compound 0.66g (55%) m.p. 179°.

Found C. 56.65, H. 4.82, N.13.06%
Requires C. 56.77, H. 4.76, N. 13.25 for $C_{15}N_{15}N_3O_5$.

Example 10

Ethyl-2[N-(3,4-dihydroxycinnamoyl)amino]pyridine-3-carboxylate

The diacetoxy compound described in Example 4 (1.92g) was reacted with methanol and sodium as described in Example 7. The crude product was crystallized from ethanol to yield pure title compound (0.85g) (56%) m.p. 178°.

Found C. 62.04, H. 4.91, N. 8.62%
Requires C. 62.19, H. 4.91, N. 8.53% for $C_{17}H_{16}N_2O_5$.

Example 11

Ethyl-2[N-(3,4-dihydroxycinnamoyl)amino]4,5,6,7-tetrahydrobenzo [b] thiophene-3-carboxylate

The diacetyl derivative described in Example 5 was suspended in methanol and treated with sodium metal as described in Example 7. Because of the low solubility of the diacetyl derivative in methanol the reaction proceeded very slowly. The mixture was warmed to 35°C when a clear solution was obtained after 0.5hr. Treatment as described in Example 7 gave the crude product (3.51g). Chromatography on Kieselgel 60 (ethyl acetate) gave title compound (2.70g) (44%) m.p. 270°.

Found C. 61.63, H. 5.49, N. 3.48, S. 8.16%
Requires C. 62.00, H. 5.46, N. 3.62, S. 8.27%.

Example 12

3-[N-3,4-dihydroxycinnamoyl)amino]-thiophene-2-carboxylic acid

The ester from Example 7 (0.85g) was treated with 1M sodium hydroxide solution (10ml) and ethanol (5ml) and stirred at room temperature for 4hr. The mixture was diluted with water and acidified with dilute hydrochloric acid. The brown solid which separated was collected, washed and dried. Recrystallization from ethyl acetate (charcoal) gave pure title compound as the mono hydrate (0.75g) m.p. 212°. Yield=88%.

Found C. 52.34, H. 4.09, N. 4.40, S. 9.73%
Requires C. 52.00, H. 4.05, N. 4.33, S. 9.92% for $C_{14}H_{11}NO_5S \cdot H_2O$.

Example 13

3-[N-(4-hydroxy-3-methoxycinnamoyl)-amino]thiophene-2-carboxylic acid.

The ester from Example 8 (0.95g) was hydrolysed as described in Example 12. The crude product was crystallized from ethanol(0.51g) (76%) m.p. 197$^{o}$.

Found C. 56.80, H. 4.37, N. 4.41, S. 9.93%
Requires C. 56.42, H. 4.10, N. 4.38, S. 10.04%

Example 14

2-[N-(3,4-dihydroxycinnamoyl)amino]pyridine 3-carboxylic acid

The ester in example 10 (0.50g) was treated with 1M Sodium Hydroxide solution and ethanol as described in Example 12. The reaction mixture was neutralized with dilute hydrochloric acid, and the precipitate which formed was collected, washed with water and dried. Treatment of this yellow-brown solid with dilute hydrochloric acid gave the title compound (0.28g) (63%) m.p. 274$^{o}$. This material could be crystallized from dimethyl formamide - water with no increase in melting point.

Found C. 56.55, H. 4.85, N. 8.65%
Requires C. 56.59, H. 4.43, N. 8.80% for $C_{15}H_{12}N_2O_5 \cdot H_2O$.

0227431

Example 15

3-[N-(3,4-Dimethoxycinnamoyl)amino]thiophene-2-carboxylic acid

The ester from Example 6 (1.50g) was treated with IM Sodium Hydroxide solution (30ml) and methanol (10ml). The reaction was stirred and warmed to 35°C. After 4hr. hydrolysis was complete and the reaction was acidified with dilute hydrochloric acid. The solid which precipitated was collected, washed and dried to yield the crude title compound (1.30g) mp 209°. Recrystallization (ethanol) gave the pure acid (1.18g)(82%) mp 214°.

Found C
Requires C. 57.65, H 4.54, N. 4.20, S. 9.62% for $C_{16}H_{15}NO_5S$

Example 16

Methyl-5-(4-chlorophenyl)-3-(3,4-diacetoxycinnamoyl-amino)-thiophene-2-carboxylate

Methyl-3-amino-5-(4-chlorophenyl)thiophene-2-carboxylate (5.35g, 0.02 mol) was dissolved in dry pyridine (50ml). A solution of the acid chloride prepared from 3,4-diacetoxycinnamic acid (5.28g, 0.02mol) and oxalyl chloride, (as described in Preparation 2), in toluene (100ml) was added dropwise with ice cooling and stirring. After stirring for a further 5hr at room temperature, the product was isolated as described in Example 1.
The title compound was a white solid mp 203$^0$ 4.79g (76%).
Found C.58.79, H.3.95, N.2.62, S.6.29%
Requires C.58.42, H.3.92, N.2.73, S.6.24% for $C_{25}H_{20}ClNO_7S$.

Example 17

t-Butyl-2-(3,4-diacetoxycinnamoylamino)-4,5,6,7-tetrahydrobenzo[b]thiophene-3-carboxylate

t-Butyl-2-amino-4,5,6,7-tetrahydrobenzo[b]thiophene-3-carboxylate described in Preparation 5 (2.53g, 0.01mol) was dissolved in dry pyridine (25ml) and a freshly prepared solution of 3,4-diacetoxycinnamoyl chloride (2.83g, 0.01mol) in dichloromethane (20ml) was added dropwise with ice cooling and stirring. After 3hr the yellow solid which had precipitated was collected, washed and dried mp 201$^o$ 2.38g (48%).
Recrystallization (EtOAc) gave pure material mp 207$^o$.

Found C.62.33, H.5.80, N.275, S.6.23%
Requires C.62.51, H.5.85, N.2.80, S.6.42% for $C_{26}H_{29}NO_7S$.

Example 18

t-Butyl-2-(3,4-diacetoxycinnamoylamino)-5-methyl-4,5,6,7-tetrahydrobenzo[b]thiophene-3-carboxylate

t-Butyl-2-amino-5-methyl-4,5,6,7-tetrahydrobenzo[b]-thiophene-3-carboxylate (8.00g, 0.03mol) as described in Preparation 6 was reacted with a freshly prepared solution of 3,4-diacetoxycinnamoyl chloride [from 3,4-diacetoxycinnamic acid (7.92g, 0.03mol)] as described in Example 17. The yellow product was collected, washed, and dried. Recrystallization (EtOAc) gave the pure title compound 4.28g (54%), mp 262°, as a mono-hydrate.
Found C.61.13, H.6.04, N.2.65, S.5.68%
Requires C.60.99, H.6.26, N.2.64, S.6.03% for $C_{27}H_{31}NO_7S.H_2O$.

Example 19

Ethyl-3-(3,4-diacetoxycinnamoylamino)pyridine-4-carboxylate

Ethyl-3-amino pyridine-4-carboxylate (2.40g, 145mmol) was dissolved in pyridine (25ml) and reacted with the acid chloride derived from 3,4-diacetoxycinnamic acid (3.82g, 145mmol) dissolved in dichloromethane as described in Example 1. Chromatography of the crude product on silica gel (EtOAc-Hexane 7:3) gave unreacted 3,4-diacetoxycinnamic acid (0.55g) and the title compound 3.16g (54%) mp 152°. A sample was recrystallized (EtOAc) and had mp 154°.

Found C.61.05, H.4.95, N.6.80

Requires C.61.16, H.4.89, N.6.75 for $C_{21}H_{20}N_2O_7$.

Example 20

Methyl-N-benzyl-3-(3,4-diacetoxycinnamoylamino)-2-carboxylate

Methyl-3-amino-N-benzylthiophene-2-carboxylate (described in Preparation 9) (3.31g), was dissolved in pyridine (40ml) and treated with the acid chloride derived from 3,4-diacetoxycinnamic acid (3.53g) dissolved in dichloromethane as described in Example 1. The crude product was a red gum 6.52g. Chromatography on silica gel ($CHCl_3$) gave recovered amine (1.50g) and title compound 3.21g (89%) mp 156°. Recrystallization (EtOAc-Hexane) gave pure title compound mp 158°.

Found C.63.29, H.4.81, N.2.77, S.6.46%

Requires C.63.27, H.4.70, N.2.84, S.6.50% for $C_{26}H_{23}NO_7S$.

Example 21

Methyl-2-(3,4-diacetoxycinnamoylamino)thiophene-3-carboxylate

Methyl-2-aminothiophene-3-carboxylate (2.60g) was dissolved in pyridine (25ml) and reacted with the acid chloride derived from 3,4-diacetoxycinnamic acid (4.00g) dissolved in dichloromethane (20ml). The reaction was carried out as described in Example 1. The crude product (5.54g) mp 155-60° was chromatographed on silica gel (EtOAc-Hexane 1:1) to yield pure title compound 3.22g (50%) mp 170°.

Found C.56.23, H.4.32, N.3.44, S.7.63%

Requires C.56.57, H.4.25, N.3.47, S.7.95% for $C_{19}H_{17}NO_7S$.

Example 22

t-Butyl-2-(3,4-dipropionoyloxycinnamoylamino)-4,5,6,7-tetrohydrobenzo[b]thiophene-3-carboxylate

t-Butyl-2-amino-4,5,6,7-tetrahydrobenzo[b]thiophene-3-carboxylate as described in Preparation 5 (2.53g, 0.01mol) was dissolved in dry pyridine (20ml) and reacted with the acid chloride derived from 3,4-dipropionoyloxycinnamic acid (2.93g) as described in Preparation 4. After 3hr the reaction mixture was poured onto ice and the solid which precipitated was collected, washed, and dried. Chromatography on silica gel ($CHCl_3$) gave 3.14g (60%) of pure title compound mp 189°.

Found C.63.59, H.6.36, N.2.81, S.6.27%

Requires C.63.74, H.6.30, N.2.66, S.6.08% for $C_{28}H_{33}NO_7S$.

Example 23

Methyl-5-(4-chlorophenyl)-3-(3,4-dihydroxycinnamoyl-amino)thiophene-2-carboxylate

The diacetoxy compound described in Example 16 (4.10g) was suspended in methanol (150ml) and treated with sodium metal (50mg) as described in Example 7. The crude product was recrystallized from methanol to yield the pure title compound 3.02g (88%) mp 256°.

Found C.58.84, H.3.77, N.3.31, Cl.8.38, S.7.11%

Requires C.58.67, H.3.75, N.3.26, Cl.8.25, S.7.46% for $C_{21}H_{16}ClNO_5S$.

Example 24

t-Butyl-2-(3,4-dihydroxycinnamoylamino)-4,5,6,7-tetrahydrobenzo[b]thiophene-3-carboxylate

The diacetoxy compound described in Example 17 (1.95g) was suspended in methanol (25ml) and treated with sodium metal as described in Example 7. The crude title compound (1.44g) mp 214-5° was recrystallized from ethylacetate (1.32g) (81%).

Found C.63.43, H.5.95, N.3.37, S.7.69%

Requires C.63,59, H.6.07, N.3.37, S.7.72% for $C_{22}H_{25}NO_5S$

Example 25

t-Butyl-2-(3,4-dihydroxycinnamoylamino)-5-methyl-4,5,6,7-tetrahydrobenzo[b]thiophene-3-carboxylate

The diacetoxy compound (0.78g) described in Example 18, was treated with methanol and sodium metal as described in Example 7. The crude product was purified by chromatography on silica gel ($CHCl_3$) to yield the pure title compound 0.32g (44%) mp 223°.

Found C.64.59, H.6.51, N.3.21

Requires C.64.31, H.6.34, N.3.26 for $C_{23}H_{27}NO_5S$.

Example 26

Methyl-2-(3,4-dihydroxycinnamoylamino)thiophene-3-carboxylate

The diacetoxy compound described in Example 21 (2.0g) was treated with methanol and sodium metal, as described in Example 7. The crude product was recrystallized from EtOAc-Hexane to yield the pure

title compound 1.27g (80%) mp 229°.

Found C.56.59, H.4.14, N.4.70, S.9.90

Requires C.56.42, H.4.10, N.4.39, S.10.02 for $C_{15}H_{13}NO_3S$.

Example 27

2-(3,4-dihydroxycinnamoylamino)-4,5,6,7-tetrahydrobenzo[b]thiophene-3-carboxylic acid

2-(3,4-Diacetoxycinnamoylamino)-4,5,6,7-tetrahydrobenzo[b]thiophene-3-carboxylic acid (1.00g) as described in Example 32 was treated with methanol and sodium metal as described in Example 7. A yellow solid mp 220° was obtained. Recrystallization from ethanol gave the pure title compound mp 224°, 0.67g (84%).

Found C.60.32, H.5.16, N.3.88, S.8.84%

Requires C.60.15, H.4.77, N.3.90, S.8.92% for $C_{18}H_{17}NO_5S$.

Example 28

Methyl-3-(3,4-dipropionoyloxycinnamoylamino)thiophene-2-carboxylate

The dihydroxy ester from Example 7 (0.50g) was treated with propionic anhydride (10ml) and conc. sulphuric acid (5 drops) and stirred at room temperature for 1hr. The mixture was poured onto ice, extracted with ethyl acetate, and the extracts were washed, dried and evaporated. The residue was crystallized for hexane when pure title compound was obtained mp 126° 0.33g (49%).

Found C.58.45, H.4.94, N.3.42, S.7.36%

Requires C.58.46, H.4.91, N.3.25, S.7.43% for $C_{21}H_{21}NO_7S$.

Example 29

t-Butyl-2-(3,4-dipropionoyloxycinnamoylamino)-5-methyl-4,5,6,7-tetrahydrobenzo[b]thiophene-3-carboxylate

The dihydroxy ester from Example 25 (0.5g) was reacted with propionic anhydride as described in Example 28. The crude product was crystallized from ethlacetate-hexane, to yield the pure title compound mp 180° 0.24g (38%).
Found C.64.37, H.6.35, N.2.59, S.5.97%
Rquires C.64.30, H.6.51, N.2.59, S.5.92% for $C_{29}H_{35}NO_7S$.
From the reaction a second product was isolated. See Example 34.

Example 30

6-Butyl-2-[3,4-di(4-methylbenzoyloxy)-cinnamoylamino]-5-methyl-4,5,6,7-tetrahydrobenzo[b]thiophene-3-carboxylate

The dihydroxy ester described in Example 25 (0.43g) was dissolved in pyridine (10ml) and stirred at room temperature. p-Toluoylchloride (0.35g, 2.1eq) was added, and the mixture was stirred for 2hr. The mixture was poured onto ice, and the yellow solid which precipitated was collected, washed, and dried to yield the crude product 0.73g mp 193°. Crystallization from Hexane-ethyl acetate gave the pure title compound 0.58g (87%) mp 203°.
Found C.70.69, H.5.91, N.2.10, S.4.70%
Requires C.70.35, H.5.99, N.2.10, S.4.82% for $C_{39}H_{39}NO_7S$.

Example 31

Methyl-3-[3,4-di(4-methylbenzoyloxy)cinnamoylamino]-thiophene-2-carboxylate

The dihydroxy ester described in Example 7 (0.50g) was reacted with p-toluoylchloride (2.1 equiv) in the manner described in Example 30. The crude product mp 170°, 0.77g, was purified by chromatography on silica gel (EtOAc-Hexane 3:7) to yield the title compound mp 185° 0.52g (60%).

Found C.66.67, H.4.46, N.2.62, S.5.65%

Requires C.67.02, H.4.54, N.2.52, S.5.77% for $C_{31}H_{25}NO_7S$.

Example 32

2-(3,4-diacetoxycinnamoylamino)-4,5,6,7-tetrahydrobenzo[b]thiophene-3-carboxylic acid

The diacetoxy ester described in Example 17 (3.20g) was treated with formic acid (100%, 50ml) and stirred at 45° for 1hr. On cooling and dilution with water, the product crystallized. Chromatography on silica gel (toluene-EtOAc 1:1) gave the pure title compound 2.35g (83%) mp 212°.

Found C.59.69, H.4.82, N.2.80, S.7.03%

Requires C.59.58, H.4.77, N.3.16, S.7.23% for $C_{22}H_{21}NO_7S$.

Example 33

2-(3,4-diacetoxycinnamoylamino)-5-methyl-4,5,6,7-tetrahydrobenzo[b]thiophene-3-carboxylic acid

The diacetoxy ester described in Example 18 (2.00g) was treated with formic acid (100%, 30ml) at 50° for 2hr.

Dilution with water gave the crude product, which was crystallized from ethyl acetate-ethanol to yield the pure title compound 0.98g (55%) mp 227°.

Found C.59.97, H.5.02, N.3.06, S.6.92%

Requires C.60.38, H.5.07, N.3.06, S.70.1% for $C_{23}H_{23}NO_7S$.

Example 34

2-(3,4-Dipropionoyloxycinnamoylamino)-5-methyl-4,5,6,7-tetrahydrobenzo[b]thiophene-3-carboxylic acid

The title compound was isolated as a by-product in Example 29. Chromatography on silica gel ($CHCl_3$) and crystallization from hexane gave the pure title compound 0.21g (37%) mp 208°.

Found C.61.83, H.5.26, N.2.88, S.6.97%

Requires C.61.84, H.5.61, N.2.89, S.6.60% for $C_{25}H_{27}NO_7S$.

Example 35

t-Butyl-2-(3,4-Dimethoxycinnamoylamino)-4,5,6,7-tetrahydrobenzo[b]thiophene-3-carboxylate

t-Butyl-2-amino-4,5,6,7-tetrahydrobenzo[b]thiophene-3-carboxylate (Preparation 5) 2.53g was dissolved in pyridine (25ml) stirred and cooled in ice. A solution of 3,4-dimethoxycinnamoyl chloride freshly prepared from 3,4-dimethoxycinnamic acid (2.08g) in dichloromethane (20ml) was added. The reaction was stirred at room temperature overnight and then processed as described in Example 1. The crude product was recrystallized from ethyl acetate to give the pure title compound 2.44g (55%) mp 177°.

Found C.64.78, H.6.64, N.3.36, S.7.11%
Requires C.64.99, H.6.59, N.3.16, S.7.23% for $C_{24}H_{29}NO_5S$.

Example 36

2-(3,4-Dimethoxycinnamoylamino)-4,5,6,7-tetrahydrobenzo[b]thiophene-3-carboxylic acid

The t-butyl ester (1.90g) described in Example 35 was treated with formic acid (100%, 25ml) and stirred at 50° for 1hr. The yellow solid which precipitated was collected, washed, and dried. Recrystallization from ethylacetate-ethanol gave the pure title compound 0.78g (48%) mp 221°.
Found C.62.11, H.5.59, N.3.72, S.8.31%
Requires C.62.00, H.5.46, N.3.62, S.8.28% for $C_{20}H_{21}NO_5S$.

By way of example, the potential therapeutic activity of illustrative compounds of this invention from the preceding Examples was demonstrated in relevant *in vivo* and *in vitro* disease models as follows:

Method A

Inhibition of Phospholipase $A_2$ Activity.

Enzyme: Phospholipase $A_2$ (Naja naja venom, Sigma)

Substrate: Diarachidoyl phosphatidylcholine (10.6mg) and α-1-palmitoyl-2-[1-$^{14}$C]-arachidonyl phosphatidylcholine (0.5μCi; 52.9mCi/mMol) in 28ml of TrisHCl buffer=100mM, pH 8.7) ultrasonicated.

Assay: Incubations containing $PLA_2$ (0.125ng) in Tris HCl buffer (100mM, pH 8.7) and $CaCl_2$ (4mM) were prepared on ice. Immediately prior to incubation at 37°C 5μl of DMSO or compound in DMSO were added. The final assay volume was 1.75ml. After 10 minutes at 37°C 750μl of substrate was added, with mixing, and the incubation continued for a further 15 min. The reaction was terminated by placing on ice and adding 5ml $CHCl_3$:MeOH (2:1v/v).

The lower organic phase was removed and stored under $N_2$ at-20°C until analysed. Analysis was by 1-dimensional t.l.c. on polygram Sil G sheets in the solvent system petroleum ether: diethyl ether: acetic acid (60:40:1,v/v). In this system the phospholipid remains at the

origin and the arachidonic acid has an approximate Rf. value of 0.36. The areas of interest were cut from the plates and radioactivity determined by liquid scintillation counting.

All compounds were tested in triplicate.

Calculation: The radioactivity of the arachidonic acid is expressed as a percentage of the total dpm cut from the plate. The percentage inhibition of arachidonic acid release compared to that of the control was calculated after correction for the level of spontaneous release where:

x = % corrected mean arachidonic acid release in control

y = % corrected arachidionic acid release after inhibition

$$\% \text{ inhibition } (z) = \frac{x-y}{x} \times 100$$

The mean of the triplicate samples was calculated giving z + standard deviation%.

Method B

## Inhibition of 5-Lipoxygenase Activity

5-Lipoxygenase enzyme was prepared as a 10,000 g supernatant from RBL-1 cells by the method of Jakschik [Jakschik, B.A., F.F. Sun, L.M. Lee, and M.M.Steinhoff,

1980, Biochem. Biophys. Res. Comm. 95, 103]. The 10,000 g supernatant was diluted with homogenization buffer to the equivalent of 1.5 - 2.5 x $10^7$ cells. $ml^{-1}$ and made 2mM with respect to $CaCl_2$. Aliquots of 0.5 ml were then dispensed into tubes, and incubated at 29$^0$C with 5 µl ethanol or compound in ethanol at the desired concentration for 2 min. Then [1-$^{14}$C] arachidonic acid was added in buffer to give a final concentration of 6.3 µM and 0.2 µCi per incubation, and the reaction continued at 29$^o$C for 2 min. The reaction was terminated by adding 1 ml of acetone and cooling on ice, 0.5 ml of ice-cold saline and 10 µl of 2N formic acid were added, and the mixture was extracted with 2 x 2 ml of chloroform. The extract was stored under $N_2$at -20$^o$C until analysis by chromatography. Activity was measured as the percentage of total radioactivity found in 5-HETE and 5,12-diHETE, and inhibition calculated as the decrease in formation of the sum of these two species in compound-treated incubates relative to control incubates.

Method C

Rat passive peritoneal anaphylaxis (PPA)

The method is essentially similar to that described previously by Ross et al (Ross, Janet W., Smith, H. and Spicer, Barbara A. Increased vascular permeability during passive peritoneal anaphylaxis in the rat. Int. Arch. Allergy appl. Immun. 51, 226, 1976.)

Animals

Charles River Sprague Dawley male rats of 225-275 g and Dunkin Hartley male white guinea pigs of 250-300 g were used.

Antiserum

Charles River Sprague Dawley male rats of 225 - 275 g were given intraperitoneal injections of 0.5 ml of either Bordetella pertussis vaccine (4 x $10^{10}$ organisms/ml; Burroughs Wellcome, London) or Pertussis vaccine absorbed (not less than 41.u. B. pertussis antigen with aluminium hydroxide; The Lister Institute Elstree, England), and subcutaneous injections of 0.5ml of an emulsion of 100 mg of ovalbumin (chicken egg albumin; crystallised and lyophilised, grade V, Sigma, London) in 2ml of isotonic saline and 3ml of incomplete Freund's adjuvant. The rats were bled by cardiac puncture on day 18, the blood was pooled and the serum separated, stored at -20°C and thawed only once before use. The serum produced 72 hour passive cutaneous anaphylaxis activity in recipient rats to a dilution of 1:64 to 1:32 which was decreased to a dilution of less than 1:2 by heating at 56°C for 4 hours.

Passive peritoneal anaphylaxis

Rats were given intraperitoneal injections of 2 ml of a 1:5 dilution of the rat anti-serum in isotonic saline. Two hours later 0.3ml of 5% solution of Pontamine Sky Blue (Raymond A. Lamb, London) in isotonic saline was injected intraveneously, followed by an intraperitoneal injection of the test compound in 1 ml of saline; (control rats received 1 ml of saline); followed 2.5 minutes later by an intraperitoneal injection of 5 ml of a Tyrode solution containing 50µg/ml heparin and 0.4 mg/ml of ovalbumin. The concentrations of the compounds were quoted as that in the 6 ml of fluid injected intraperitoneally. Exactly 5 minutes after

challenge the rats were stunned and bled and their peritoneal fluids were collected by opening their peritoneal cavities over funnels into polycarbonate tubes in ice. The supernatants were separated from the cellular residue by centrifuging at 150 g for 5 minutes and any samples obviously contaminated with blood were discarded for estimation of dye, histamine and SRS-A. Groups of at least 5 rats were used for each dose of compound and the treatments were randomized.

Assay of peritoneal fluids

Collected peritoneal fluids were centrifuged and the supernatant fluids assayed for dye immediately. 0.5 ml samples of the supernatants were added to 1 ml of 12% trichloracetic acid and stored at -20$^{o}$C and used to assay for histamine. The remainders of the supernatant fluids were placed in a boiling water bath for 5 minutes and stored frozen at -20$^{o}$C until assayed for SRS-A.

Dye Assay

The optical densities (OD) of the supernatants which were not contaminated with blood were determined immediately at 625nm.

Histamine Assay

Histamine was assayed using an automated spectrofluorimetric system (technicon Autoanalyser) by a method similar to that of Evans, D.P., Lewis, J.A. and Thomson, D.S.: (An automated fluorimetric assay for the rapid determination of histamine in biological fluids. Life Sci. 12, 327, 1973). At the concentrations used the compounds tested did not interfere with the assay.

SRS-A Assay

SRS-A was assayed on the isolated guinea pig ileum preparation in the presence of atropine (5 x $10^{-7}$M) and mepyramine maleate ($10^{-6}$M), the latter to abolish the histamine response. (Brocklehurst, W.E. The release of histamine and formation of a slow reacting substance (SRS-A) during anaphylactic shock. J. Physiol., Lond. 151, 416, 1960). Bulked peritoneal fluids from passively sensitised and challenged rats were centrifuged, heated, stored at -20°C in 0.5 ml aliquots, and used as a reference SRS-A standard, and arbitrarily designated as containing 10 Units per ml. Concentrations of the unknown were bracketed by reference SRS-A samples. At the concentrations used, the compounds tested did not interfere with the assay.

Method D

Catabolin (Pig Interleukin-1)-Induced Breakdown of Porcine Articular Cartilage in vitro

This model of cartilage degradation is adapted from the biological assay for catabolin of J. Dingle, et al. (Biochem. J. 184, 177-180, 1979).

Catabolin (pig interleukin-1, IL-1) is identified by its ability to induce the breakdown of cartilage proteoglycans in living but not in killed cartilage.

The effect of catabolin on proteoglycan breakdown is measured by the release of $^{35}SO_4$, precipitable by cetlpyridinium chloride (CPC), from pieces of pre-labelled porcine articular cartilage.

The cartilage is obtained from the metacarpophalangeal joints of freshly killed young pigs; it is trimmed, washed and, after an overnight incubation, labelled with 10 µCi/ml/4 cartilage slices $Na_2{}^{35}SO_4$ for 24 hours. The culture medium used is Dulbecco's modification of Eagle's medium (DMEM) containing 10% foetal calf serum (to inhibit direct enzymic degradation of the cartilage), glutamine and antibiotics. The labelled cartilage is washed (DMEM+1 mg/ml unlabelled $Na_2SO_4$) and cultured in multiwell dishes, containing 0.5 ml of either control culture medium or medium containing catabolin (derived from either porcine synovial tissue or from porcine mononuclear leukocytes), at 36.5°C in a humidified atmosphere of 5% $CO_2$ in air. Compounds are generally dissolved in dimethyl sulphoxide (DMSO) and added to both control and catabolin groups (n=4). Killed cartilage slices (frozen and thawed three times in liquid $N_2$) are also cultured to confirm the lack of any direct enzymic degradation of the cartilage.

The culture medium is changed every 2 days for 6 days and the culture medium from the 3 collection periods is pooled. At the end of the experiment the residual cartilage slices are digested overnight in papain (5 mg/ml in 50 mM phosphate buffer, pH 6.5) and samples of the cartilage-conditioned medium are treated with CPC (5% in 0.3 M KCl) which precipitates the radiolabelled glycosoaminoglycan products of catabolin-induced degradation.

Aliquots of the cartilage-conditioned medium, the CPC treated medium and the cartilage digests are assayed by liquid scintillation counting.

The release of $^{35}SO_4$ proteoglycan from the cartilage is determined from the CPC-precipitable label released into the medium as a percentage of the total label initially incorporated into the cartilage, i.e.

% proteoglycan breakdown =

$$\frac{\text{CPC precipitable label released}}{\text{total label released into medium + Label in residual cartilage digest.}} \times 100$$

Subtraction of the release in control groups from that in catabolin-treated groups gives the breakdown actually induced by catabolin.

Compound activity is quoted as % inhibition of this catabolin-induced cartilage breakdown.

Compound toxicity is assessed by measurement of the incorporation of $^{35}SO_4$ into proteoglycan by cartilage cultured in the presence of compound, after a second 4-day period of culture in the absence of compound.

## BIOLOGICAL RESULTS

### 1. Enzyme Inhibition

| Example No. | 5-Lipoxygenase Inhibition at 20μM (%) | Phospholipase $A_2$ Inhibition at 20μM (%) |
|---|---|---|
| 6 | 81 | 31 |
| 7 | 93 | 84 |
| 8 | 98 | 26 |
| 9 | 97 | 71 |
| 10 | 95 | 10 |
| 11 | 91 | 92 |
| 12 | 88 | 36 |
| 13 | 64 | 26 |
| 14 | 67 | - |
| 15 | 0 | 10 |
| 16 | 60 | - |

### 2. Rat Passive Peritoneal Anaphylaxis

All compounds were injected i.p. to give a final concentration of $2 \times 10^{-4}$M with a contact time of 2½ minutes.

| Example No. | Percentage Inhibition Dye | Histamine | SRS-A |
|---|---|---|---|
| 7 | 19 | 71 | 69 |
| 9 | 41 | 65 | 45 |
| 10 | 29 | 62 | 62 |
| 11 | -8 | 29 | 57 |
| 12 | 43 | 72 | 49 |
| 13 | 24 | 73 | 59 |
| 14 | 54 | 89 | 72 |
| 15 | 43 | 74 | 50 |

3. Catabolin (Pig Interleukin-1)-Induced Breakdown of Porcine Articular Cartilage in vitro

| Example No. | | % Inhibition catabolin -induced breakdown |
|---|---|---|
| 7 | 10 µg/ml | 19** |
| | 3 µg/ml | -29 |

<u>Claims</u>



1. A compound of formula (I):

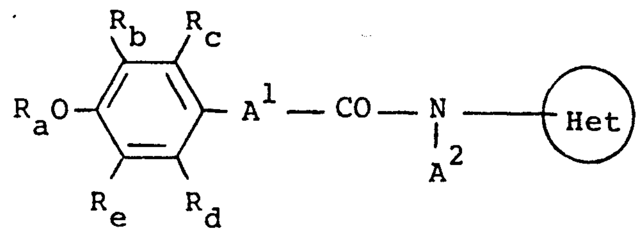

(I)

or a salt, ester, amide or solvate thereof, characterised in that $R_a$ represents hydrogen, substituted or unsubstituted alkyl or an acyl group; $R_b$ and $R_c$ are independently hydrogen or a substituted or unsubstituted aryl group or a group $-OR_a$; or $R_b$ and $R_c$ together with the carbon atoms to which they are attached form a substituted or unsubstituted carbocyclic group;
$R_d$ and $R_e$ are independently hydrogen, substituted or unsubstituted aryl or a group $-OR_a$; or $R_d$ and $R_e$ together with the carbon atoms to which they are attached form a substituted or unsubstituted carbocyclic group;
$A^1$ represents an alkylene or an alkenylene group;
$A^2$ represents hydrogen, substituted or unsubstituted alkyl or an aralkyl group substituted or unsubstituted in the aryl moiety;

and Het represents a substituted or unsubstituted heterocyclic group.

2. A compound as claimed in claim 1, wherein the heterocyclic group Het represents a group of formula:

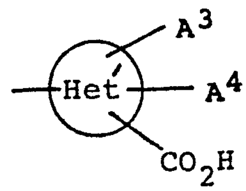

wherein Het′ represents a 5- or 6- membered single ring having up to 4 hetero atoms in the ring selected from oxygen, nitrogen or sulphur; and

$A^3$ and $A^4$ are independently hydrogen, alkyl, substituted or unsubstituted aryl or $A^3$ and $A^4$ together with the atoms to which they are attached form a saturated or unsaturated carbocyclic group.

3. A compound as claimed in any one of claims 1 or 2, represented by the general formula (II):

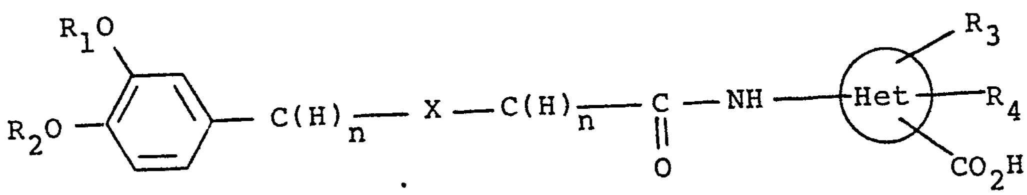

(II)

or a salt, ester, amide or solvate thereof,

in which

Het is as defined above in relation to formula (I),

either n is 1 and X is a double bond,

or n is 2 and X is a single bond,

$R_1$ and $R_2$ are indendently hydrogen, $C_1$-$C_6$ alkyl or acyl, $R_3$ and $R_4$ are independently hydrogen, $C_1$-$C_6$ alkyl, substituted or unsubstituted aryl, or $R^3$ and $R^4$,

together with the atoms to which they are attached, form a saturated or unsaturated carbocyclic ring.

4. A compound as claimed in claim 3, wherein Het' is thiophene.

5. A compound as claimed in claim 3 or claim 4, wherein $R_3$ and $R_4$ are suitably hydrogen; or $R_3$ and $R_4$, together with the atoms to which they are attached, from a fused benzo, tetrahydrobenzo or cyclopentyl ring.

6. A compound as claimed in claim 1, selected from the list consisting of:

methyl 3-[N-(3,4-dihydroxycinnamoyl)-amino]thiophene-2-carboxylate; and

ethyl-2[N-(3,4-dihydroxycinnamoyl)amino]4,5,6,7-tetrahydrobenzo [b] thiophene-3-carboxylate; or a pharmaceutically acceptable salt, ester, amide or solvate thereof.

7. A process for preparing a compound of formula (I), as defined in claim 1, or a pharmaceutically acceptable salt, ester, amide or solvate thereof, which process comprises reacting a compound of formula (III):

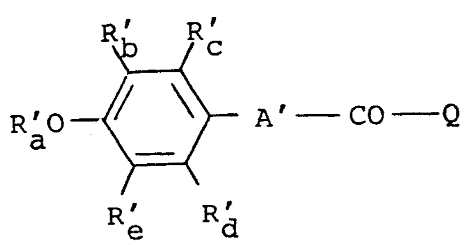

(III)

in which $R'_a$, $R'_b$, $R'_c$, $R'_d$ and $R'_e$ are either $R_a$, $R_b$, $R_c$, $R_d$ or $R_e$ as defined in respect of formula (I) or a group or atom convertible to $R_a$, $R_b$, $R_c$, $R_d$, or $R_e$; $A^1$ is as defined in relation to formula (I) and Q is a leaving group; with a compound of formula (IV)

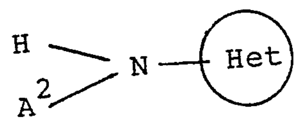

(IV)

in which Het is as defined in respect of formula (I);

to form a compound of formula (Ia)

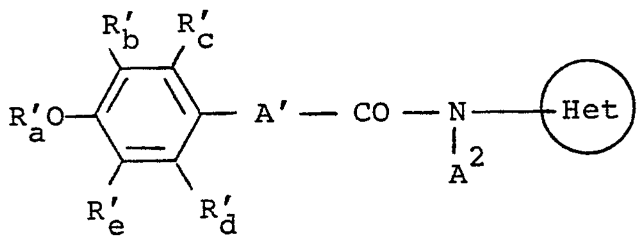

(Ia)

and thereafter where appropriate converting $R'_a$, $R'_b$, $R'_c$, $R'_d$ or $R'_e$ to $R_a$, $R_b$, $R_c$, $R_d$ or $R_e$ respectively, and optionally forming a salt, ester, amide or solvate thereof.

8. A pharmaceutical composition comprising a pharmaceutically acceptable compound of the formula (I) as defined in claim 1, or a pharmaceutically acceptable salt, ester, amide or solvate thereof, in admixture or conjunction with a pharmaceutically acceptable carrier.

9. A pharmaceutically acceptable compound of formula (I), as defined in claim 1, or a pharmaceutically

acceptable salt, ester, amide or solvate thereof for use as an active therapeutic substance.

10. A pharmaceutically acceptable compound of formula (I), or a pharmaceutically acceptable salt, ester, amide or solvate thereof, for use in the treatment or prophylaxis of allergic diseases, such as asthma, hayfever, rhinitis or allergic eczema, or for the treatment of inflammatory conditions such as arthritis and psoriasis.

11. The use of a pharmaceutically acceptable compound of formula (I) as defined in claim 1, or a pharmaceutically acceptable salt, ester, amide or solvate thereof, for the manufacture of a medicament for the treatment or prophylaxis of allergic diseases, such as asthma, hayfever, rhinitis or allergic eczema, or for treatment of inflammatory conditions such as arthritis and psoriasis.

European Patent Office

# EUROPEAN SEARCH REPORT

0227431

Application number

EP 86 30 9873

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 90, no. 17, 23nd April 1979, page 501, abstract no. 137683c, Columbus, Ohio, US; & ES-A-457 478 (LABORATORIO FARMACEUTICO QUIMICO "LAFARQUIM" S.A.) 16-08-1978<br>* Abstract *<br>--- | 1,7,11 | C 07 D 333/38<br>C 07 D 333/68<br>C 07 D 231/40<br>C 07 D 213/79<br>C 07 D 213/80<br>A 61 K 31/38<br>A 61 K 31/415<br>A 61 K 31/44 |
| X | DE-A-2 328 391 (DEGUSSA)<br>* Page 20, example 9; claims *<br>--- | 1,7-11 | |
| Y | CHEMICAL ABSTRACTS, vol. 88, no. 21, 22nd May 1978, page 597, abstract no. 152482p, Columbus, Ohio, US; B. DRAGOMIR et al.: "New semisynthetic penicillins obtained by coupling 6-aminopenicillanic acid with cinnamic acid derivatives", & REV. MED.-CHIR. 1977, 81(3), 489-94<br>* Abstract *<br>--- | 1,7,11 | TECHNICAL FIELDS SEARCHED (Int. Cl.4) |
| Y | CHEMICAL ABSTRACTS, vol. 88, no. 23, 5th June 1978, pages 597-598, abstract no. 170172s, Columbus,. Ohio, US; & ES-A-444 346 (LABORATORIOS CUSI S.A.) 01-10-1977<br>* Abstract *<br>----- | 1,7 | C 07 D 333/00<br>C 07 D 231/00<br>C 07 D 213/00<br>A 61 K 31/00 |

The present search report has been drawn up for all claims

| Place of search | Date of completion of the search | Examiner |
|---|---|---|
| THE HAGUE | 24-02-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document